# EUROPEAN PATENT APPLICATION

(11) **EP 1 479 400 A1**
(43) Date of publication of application: **24.11.2004**
(21) Application number: 03425331.0
(22) Date of filing: 22.05.2003
(51) Int. Cl.: A61L 9/12, A61L 9/04, B60H 3/00, A01M 1/20, A01N 25/04

(54) **Device for dispensing deodorizing or fragrancing or sanitizing substances or the like for enclosed spaces, particularly for the cabin of motor vehicles**

(71) Applicant: Deoflor S.p.A., 27030 Confienza (Pavia) (IT)
(72) Inventor: Ruffina, Laura, 27036 Mortara (IT)
(74) Representative: Modiano, Guido, Dr.-Ing.

(57) **Abstract**

A device for dispensing deodorizing or fragrancing or sanitizing substances or the like for enclosed spaces, particularly for the cabin of motor vehicles, which comprises a hollow container (2) in which there is a compartment (3) that accommodates a deodorizing or fragrancing or sanitizing product or the like (4); the product (4) comprises a carrier in gel form that is substantially solid and preferably transparent or translucent and contains a volatile active substance; the container (2) is provided with engagement means (8), which can be associated with an air vent of the enclosed space, and has inlets (10) for the inflow of ventilation air into the compartment (3) and air outlets (11) that connect the compartment (3) to the enclosed space so as to obtain, as a consequence of the stream of air that passes through the compartment (3) in which the deodorizing or fragrancing or sanitizing product or the like (4) is placed, the diffusion of the volatile active substance into the enclosed space.

## Description

The present invention relates to a device for dispensing deodorizing or fragrancing or sanitizing substances or the like for enclosed spaces, particularly for the cabin of motor vehicles.

Devices for dispensing deodorizing or fragrancing substances into the cabin of motor vehicles are known.

One type of these devices comprises a plastics container, which is designed to be applied to the dashboard or to other areas of the vehicle by means of adhesive and in which a solid fragrancing or deodorizing product is inserted. Devices of this type have the drawback that the adhesive can fail or mark the dashboard or the surface to which it is applied. Moreover, in this type of device it is impossible or particularly complicated to replace the deodorizing or fragrancing product once it is used up.

Another type of device for deodorizing and/or fragrancing the cabin of motor vehicles comprises a solid substrate, made of cardboard or wood or plastics, which is impregnated with deodorant or fragrance and can be hung from the rear part of the rearview mirror located inside the cabin of the motor vehicle. These devices have the disadvantage that they deplete unevenly, with rapid deterioration of the deodorizing or fragrancing effect. Furthermore, this type of device is scarcely appreciated from an aesthetic standpoint.

Another type of device for fragrancing or deodorizing the cabin of motor vehicles comprises a container in which there is a deodorizing or fragrancing liquid product, which is diffused into the enclosed space through a semipermeable membrane made of polymeric material. This type of device is fairly effective, but due to its very structure it does not allow to obtain particularly pleasant aesthetic effects.

Another type of device for fragrancing and/or deodorizing the cabin of motor vehicles comprises a container in which there is a reservoir, generally made of glass, which contains a deodorizing or fragrancing product in the liquid state. A wick is dipped in the fragrancing and/or deodorizing product and is exposed to the air in a region of the container. The container has engagement means that can be associated with the grilles of the air vents, and has openings arranged so that the stream dispensed by the air vents passes through the region of the container at which the wick dipped in the liquid product is arranged. This type of device is fairly effective and aesthetically appreciated, but it has the drawback of using a reservoir that can accidentally detach from the container that supports it and fall, with the danger of breakage of the reservoir or in any case with the consequence of leakage of the fragrancing and/or deodorizing product, which inevitably damages the upholstery of the motor vehicle.

Another type of device is constituted by a solid transparent substrate provided with recesses in which the fragrancing and/or deodorizing product is arranged. These devices can be hung from the rear part of the rearview mirror or can be applied to the dashboard by means of adhesive. This type of device, too, has the drawback that its aesthetics is not particularly appreciated by many users, its dispensing is not adjustable, and if applied to the dashboard or other parts of the motor vehicle by means of an adhesive has the problem that the device can separate accidentally and the adhesive can dirty or damage the surface to which it is applied.

The aim of the present invention is to provide a device for dispensing deodorizing or fragrancing or sanitizing substances or the like for enclosed spaces, particularly for the cabin of motor vehicles, that is capable of obviating the drawbacks described above with reference to devices of the conventional type.

Within this aim, an object of the invention is to provide a device that ensures optimum effectiveness in releasing deodorizing or fragrancing or sanitizing substances or the like and can be particularly pleasant aesthetically.

A further object of the invention is to provide a device that even in case of accidental falls safely avoids the possibility of damage to the upholstery of the motor vehicle.

A still further object of the invention is to provide a device in which it is possible to control and adjust the release of the deodorizing or fragrancing or sanitizing substance or the like.

Another object of the invention is to provide a device in which it is possible to replace the deodorizing or fragrancing or sanitizing product or the like very easily and simply when it is used up.

This aim and these and other objects that will become better apparent hereinafter are achieved by a device for dispensing deodorizing or fragrancing or sanitizing substances or the like for enclosed spaces, particularly for the cabin of motor vehicles, characterized in that it comprises a hollow container in which there is a compartment that accommodates a deodorizing or fragrancing or sanitizing product or the like, which comprises a carrier in gel form that contains a volatile active substance, said container being provided with engagement means that can be associated with an air vent of the enclosed space and having at least one opening for the inflow of ventilation air in said compartment and at least one outlet for the air that connects said compartment to the enclosed space.

Further characteristics and advantages of the invention will become better apparent from the description of a preferred but not exclusive embodiment of the device according to the invention, illustrated by way of non-limitative example in the accompanying drawings, wherein:

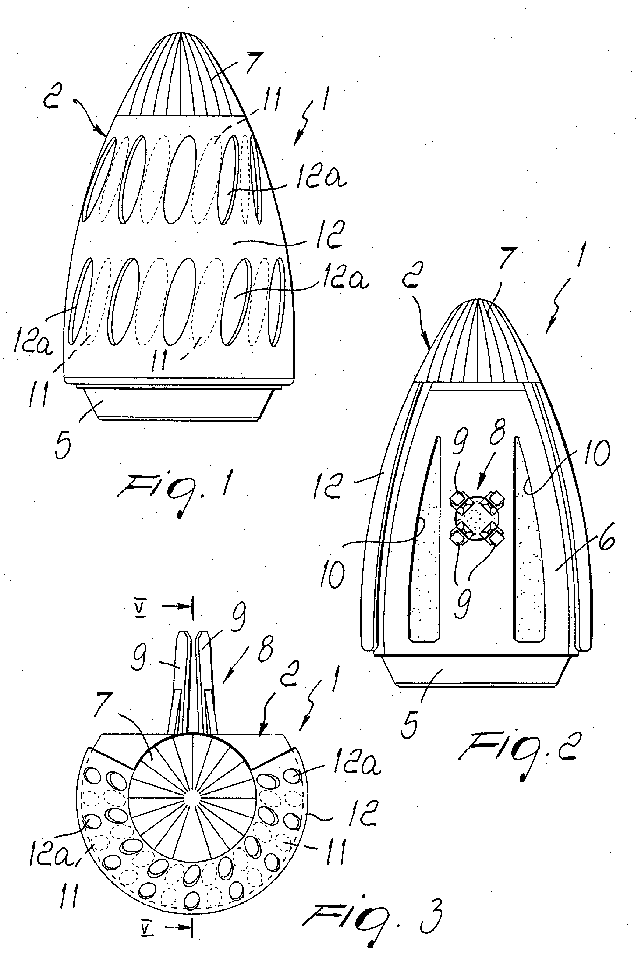

Figure 1 is a front elevation view of the device according to the invention;
Figure 2 is a rear elevation view of the device according to the invention;
Figure 3 is a top plan view of the device according to the invention;

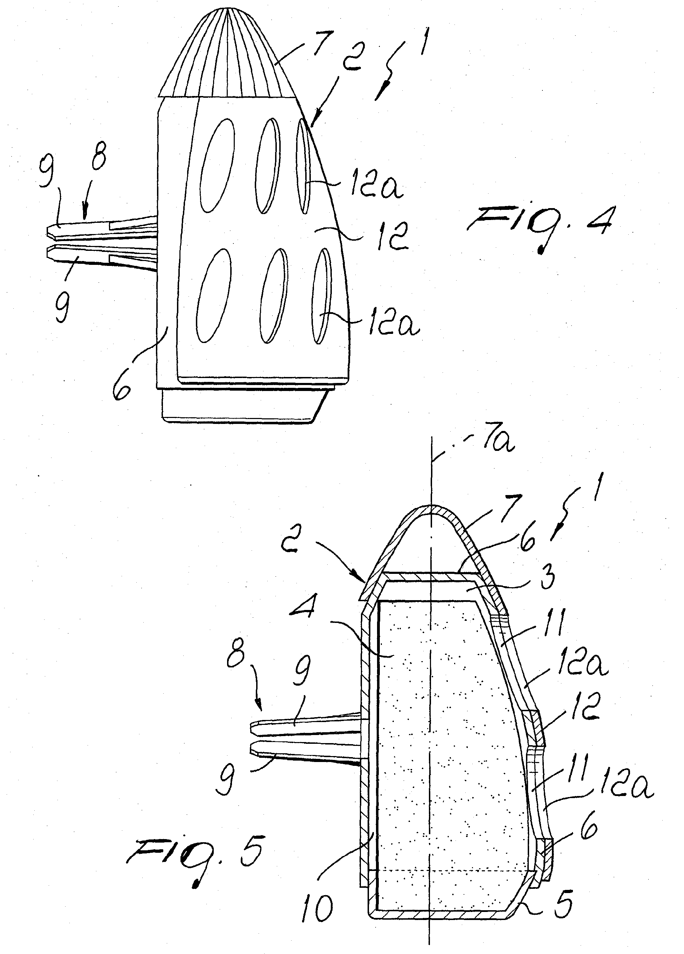

Figure 4 is a side elevation view of the device according to the invention;
Figure 5 is a sectional view of Figure 3, taken along the line V-V.

With reference to the figures, the device according to the invention, generally designated by the reference numeral 1, comprises a container 2, which is preferably made of plastics by molding.

The container 2 is hollow, so as to form internally a compartment 3 that accommodates a deodorizing or fragrancing or sanitizing product or the like 4.

Preferably, the compartment 3 is closed in a downward region by a bottom 5, which is detachably connected to the body 6 of the container 2.

In an upper region of the container 2 there is a knob 7, which is associated, so that it can rotate about an axis 7a, with the body 6 of the container 2, as will become better apparent hereinafter.

The container 2 is provided with engagement means 8, which are associable with the grille of an air vent of the enclosed space, in particular to the air vent of the cabin of a motor vehicle. The engagement means can be constituted by wings 9, which can be formed advantageously monolithically with the body 6 of the container 2 and can engage with a snap action, by using their elastic flexibility, with the grille of the air vent.

The container 2 has at least one inlet 10 for the ventilation air that arrives from the air vent and at least one outlet 11 for the air that connects the compartment 3 to the surrounding enclosed space.

In particular, preferably there are multiple air inlets 10 arranged on the side of the body 6 of the container 2 that faces the air vent, so that the air stream delivered by the air vent is conveyed adequately into the compartment 3.

There are also multiple outlets 11, which are distributed on the surface of the body 6 of the container 2 so as to achieve a good diffusion of the air stream that flows through the compartment 3 into the enclosed space that surrounds the device according to the invention.

Conveniently, there are means for adjusting the extent of the opening of the inlets 10 and/or outlets 11 so as to control and adjust the quantity of deodorizing or fragrancing or sanitizing substance or the like released by the product.

The adjustment means comprise a movable element 12 of the container 2, for example a partition with holes 12a, which is arranged outside the body 6 of the container 2 proximate to the outlets 11, as shown, or proximate to the inlets 10, and is connected to the knob 7 so that a rotation of the knob 7 about its own axis 7a with respect to the body 6 of the container 2 closes at least partially the openings 10 or 11 or clears the openings completely.

As an alternative, the movable element 12 can be arranged internally instead of on the outside of the body 6 of the container 2.

Preferably, the container 2 is made at least partially of transparent or translucent material, so as to allow to view the deodorizing or fragrancing or sanitizing product or the like 4, present in the compartment 3, from the outside of the container 2.

On the outer surface of the container 2 it is possible to provide patterns, optionally in relief, or it is possible to apply labels or other elements in order to improve the aesthetic effect of the container 2.

According to the invention, the deodorizing or fragrancing or sanitizing product or the like 4 comprises a carrier in gel form, which contains a volatile active substance.

The gel that constitutes the carrier is preferably substantially transparent or translucent, so as to cooperate with the transparent or translucent areas of the container 2 in order to obtain a particular optical effect.

Preferably, the gel that constitutes the carrier is substantially solid.

Preferably, the product 4 is formulated so as to be self-supporting.

The gel that constitutes the carrier can be constituted by a synthetic polymer, such as for example a polyacrylate or a polyurethane or ethylene vinyl acetate, or by a water-soluble or anhydrous polymer.

Preferably, the carrier is constituted by a base that comprises a mixture of aliphatic hydrocarbons, hydrogen-treated paraffins, long-chain alcohols, butylhydroxytoluene, mineral oils and gelling agents.

In this case, the carrier has a density of more than 1000 kg/m³, a flash point of over 350 °F and a melting point of more than 90 °C.

A carrier of this type incorporates a volatile active substance in a percentage that is substantially comprised between 3 and 30% by weight relative to the overall weight of the deodorizing or fragrancing or sanitizing product or the like 4, and such percentage is preferably substantially comprised between 10 and 20%.

As an alternative, the carrier can also be constituted by a base that is constituted by polyamide resins such as ATPA or ETPA. The carrier has an average viscosity of 600-800 cps/mPa.s at 170 °C, a softening point between 80 and 105 °C, and a flash point higher than 290 °C.

A carrier of this kind incorporates a volatile active substance in a percentage substantially comprised between 5 and 60% by weight relative to the overall weight of the deodorizing or fragrancing or sanitizing product or the like, and said percentage is preferably substantially comprised between 30 and 50%.

The volatile active substance, for both of the preferred carriers of the type described above, can be constituted by a fragrance and/or a deodorant and/or a bactericidal/sanitizing product and/or an insecticide and/or insect-repellent substance.

The product 4, constituted by the carrier and the active substance, is substantially stable in a temperature range that is substantially comprised between -10 °C and 80 °C.

The product 4, due to its particular formulation, is practically free from syneresis phenomena.

The product 4 can be poured directly into the container 2 or into a preform to be inserted subsequently, at least partially, in the container 2.

For example, the product 4 can be poured directly onto the bottom 5 and marketed together with the bottom 5 in order to constitute a refill of the device. As an alternative, the product 4 can be poured into a bottom or other open or openable container to be arranged in the compartment 3 of the container 2.

Also as an alternative, the product 4 can be partially coated with film-forming polymeric agents, so as to directly constitute, with the part covered by said film-forming polymeric agents, a portion of the container 2.

Optionally, the preform into which the product 2 is poured can be constituted by a portion to be assembled to the container 2 or in any case to be inserted, together with the product, inside the compartment 3, and by a part that is designed to be removed in order to allow to expose the product 4 to the air.

Conveniently, it is possible to embed in the product 4 optically detectable additives or corpuscles that can be constituted by glitter, bodies made of synthetic material such as spheres or other geometric shapes, pearlescent colors, metallic pigments, optical brighteners, or other elements suitable to produce an optically pleasant effect for the product.

Merely by way of indication, a few examples of preparation of the product 4 to be introduced in the container 2 of the device according to the invention are given hereafter.

| EXAMPLE 1 | |
|---|---|
| *Ingredient* | % |
| Fragrance 055200737 | 20 |
| Versagel F-1100 | 70 |
| Mineral oil | 10 |
| Dye | traces |
| Versagel F-1100 is supplied by Penreco | |
| Fragrance 055200737 is supplied by Sensient Fragrances | |

| EXAMPLE 2 | |
|---|---|
| *Ingredient* | % |
| Fragrance 055200733 | 20 |
| Versagel F-1000 | 80 |
| Versagel F-1000 is supplied by Penreco | |
| Fragrance 055200733 is supplied by Sensient Fragrances | |

| EXAMPLE 3 | |
|---|---|
| *Ingredient* | % |
| Fragrance AD 9115 | 50 |
| Sylvagel 1000 | 50 |
| Dye | traces |
| Sylvagel 1000 is supplied by Arizona Chemical | |
| Fragrance AD 9115 is supplied by Quest | |

| EXAMPLE 4 | |
|---|---|
| *Ingredient* | % |
| Fragrance AD 9118 | 40 |
| Sylvaclear A200 | 50 |
| Mineral oil | 10 |
| Sylvaclear A200 is supplied by Arizona Chemical | |
| Fragrance AD 9118 is supplied by Quest | |

In practice it has been found that the device according to the invention fully achieves the intended aim, since it can have different aesthetic configurations capable of meeting the most disparate requirements of the user, ensures gradual release of the active substance into the enclosed space, offers adequate assurances against the danger of dirtying in case of separation from the air vent, and is very easy to use.

The device according to the invention as a whole is further capable of withstanding, without altering, the temperatures usually reached in the cabin of a motor vehicle.

The device thus conceived is susceptible of numerous modifications and variations, all of which are within the scope of the inventive concept; all the details may further be replaced with other technically equivalent elements.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device for dispensing deodorizing or fragrancing or sanitizing substances or the like for enclosed spaces, particularly for the cabin of motor vehicles, **characterized in that** it comprises a hollow container in which there is a compartment that accommodates a deodorizing or fragrancing or sanitizing product or the like, which comprises a carrier in gel form that contains a volatile active substance, said container being provided with engagement means that can be associated with an air vent of the enclosed space and having at least one opening for the inflow of ventilation air in said compartment and at least one outlet for the air that connects said compartment to the enclosed space.

2. The device according to claim 1, **characterized in that** said product is substantially transparent or translucent.

3. The device according to claim 1, **characterized in that** said gel is substantially solid.

4. The device according to one or more of the preceding claims, **characterized in that** said product is self-supporting.

5. The device according to one or more of the preceding claims, **characterized in that** said carrier is constituted by a polymer.

6. The device according to one or more of the preceding claims, **characterized in that** said carrier is constituted by a polyamide resin such as ATPA or ETPA.

7. The device according to one or more of the preceding claims, **characterized in that** said carrier is a polyurethane.

8. The device according to one or more of the preceding claims, **characterized in that** said carrier is made of ethylene vinyl acetate.

9. The device according to one or more of the preceding claims, **characterized in that** said gel is constituted by a base that comprises a mixture of aliphatic hydrocarbons, paraffins treated with hydrogen, long-chain alcohols, butylhydroxytoluene, mineral oils and gelling agents.

10. The device according to one or more of the preceding claims, **characterized in that** said product has a softening temperature of more than 80 °C.

11. The device according to one or more of the preceding claims, **characterized in that** said product is substantially stable over a temperature range substantially comprised between -10 °C and 80 °C.

12. The device according to one or more of the preceding claims, **characterized in that** optically detectable additives or corpuscles are incorporated in said product.

13. The device according to one or more of the preceding claims, **characterized in that** said additives or corpuscles are constituted by at least one element selected among: glitter, bodies made of synthetic material, pearlescent colors, metallic pigments, optical brighteners.

14. The device according to one or more of the preceding claims, **characterized in that** said volatile active substance is present in said product in a percentage that is substantially comprised between 3% and 60% by weight with reference to the overall weight of the product.

15. The device according to one or more of the preceding claims, **characterized in that** said volatile active substance is present in said product in a percentage that is substantially comprised between 10 and 50% by weight relative to the overall weight of the product.

16. The device according to one or more of the preceding claims, **characterized in that** said volatile active substance is constituted by a fragrance and/or a deodorant and/or a bactericidal/sanitizing product and/or an insecticide and/or an insect-repellent substance.

17. The device according to one or more of the preceding claims, **characterized in that** said product is partially coated with film-forming polymeric agents.

18. The device according to one or more of the preceding claims, **characterized in that** said product is poured directly into said compartment of the container.

19. The device according to one or more of the preceding claims, **characterized in that** said product is poured into a supporting preform.

20. The device according to one or more of the preceding claims, **characterized in that** said preform is detachably associable with said container.

21. The device according to one or more of the preceding claims, **characterized in that** said container is made at least partially of transparent or translucent material, in order to allow the viewing of said product from the outside of said container.

22. The device according to one or more of the preceding claims, **characterized in that** it comprises means for adjusting the extent of the opening of said inlets and/or outlets.

23. The device according to one or more of the preceding claims, **characterized in that** said adjustment means comprise a knob that can be operated and is associated with an element that is associated with said body of the container and can move, by way of the action of said knob, in order to close at least partially or clear completely said inlets and outlets.

24. The device according to one or more of the preceding claims, **characterized in that** said engagement means comprise tabs of said container that engage by snap action the grille of an air vent of the interior of motor vehicles.
